# EUROPEAN PATENT SPECIFICATION

(11) **EP 4 261 540 B1**
(45) Date of publication and mention of the grant of the patent: **17.12.2025**
(21) Application number: 22738330.4
(22) Date of filing: 05.01.2022
(51) Int. Cl.: G01N 33/68

(54) **MARKER FOR ASSISTING IN DIAGNOSIS OF NEPHROTIC SYNDROME AND USE THEREOF**
MARKER ZUR UNTERSTÜTZUNG DER DIAGNOSE DES NEPHROTISCHEN SYNDROMS UND VERWENDUNG DAVON
MARQUEUR D'AIDE AU DIAGNOSTIC DU SYNDROME NÉPHROTIQUE ET SON UTILISATION

(30) Priority: 14.01.2021 JP 2021004184
(43) Date of publication of application: 18.10.2023
(73) Proprietor: Niigata University, Niigata-shi, Niigata 950-2181 (JP)
(72) Inventor: KAWACHI Hiroshi, Niigata-shi, Niigata 951-8510 (JP)
(74) Representative: Cabinet Becker et Associés
(86) International application number: PCT/JP2022/000138
(87) International publication number: WO 2022/153907

(56) References cited:
- WO-A2-2010/019565
- JP-A- 2018 059 877
- SAYURI TAKAMURA ET AL: "TH-PO069 Par6[beta] Interaction with Ephrin-B1 at the Slit Diaphragm Could Be a Differential Diagnostic Marker of Nephrotic Syndrome: Interaction of the Par-Complex Molecules with Ephrin-B1/Nephrin in Podocyte", vol. 28, no. Suppl. 1, 1 January 2017 (2017-01-01), pages 121, XP009538244, ISSN: 1046-6673, Retrieved from the Internet <URL:https://www.asn-online.org/education/kidneyweek/archives/KW17Abstracts.pdf>
- FUKUSUMI YOSHIYASU, ZHANG YING, YAMAGISHI RYOHEI, ODA KANAKO, WATANABE TORU, MATSUI KATSUYUKI, KAWACHI HIROSHI: "Nephrin-Binding Ephrin-B1 at the Slit Diaphragm Controls Podocyte Function through the JNK Pathway", JOURNAL OF THE AMERICAN SOCIETY OF NEPHROLOGY, vol. 29, no. 5, 1 May 2018 (2018-05-01), US , pages 1462 - 1474, XP055950601, ISSN: 1046-6673, DOI: 10.1681/ASN.2017090993
- TAKAMURA SAYURI, FUKUSUMI YOSHIYASU, ZHANG YING, NARITA ICHIEI, KAWACHI HIROSHI: "Partitioning-Defective-6–Ephrin-B1 Interaction Is Regulated by Nephrin-Mediated Signal and Is Crucial in Maintaining Slit Diaphragm of Podocyte", THE AMERICAN JOURNAL OF PATHOLOGY, vol. 190, no. 2, 1 February 2020 (2020-02-01), US , pages 333 - 346, XP055950606, ISSN: 0002-9440, DOI: 10.1016/j.ajpath.2019.10.015
- FUKUSUMI YOSHIYASU, NAOKO MIYAUCHI, TAEKO HASHIMOTO, AKIRA SAITO, HIROSHI KAWACHI: "Therapeutic target for nephrotic syndrome: Identification of novel slit diaphragm associated molecules", WORLD JOURNAL OF NEPHROLOGY, vol. 3, no. 3, 1 January 2014 (2014-01-01), pages 77 - 84, XP055950609, ISSN: 2220-6124, DOI: 10.5527/wjn.v3.i3.77
- HIROSHI KAWACHI ; KOICHI SUZUKI ; NAOKO MIYAUCHI ; TAEKO HASHIMOTO ; YASUHIRO OTAKI ; FUJIO SHIMIZU: "Slit diaphragm dysfunction in proteinuric states: identification of novel therapeutic targets for nephrotic syndrome", CLINICAL AND EXPERIMENTAL NEPHROLOGY, vol. 13, no. 4, 7 March 2009 (2009-03-07), To , pages 275 - 280, XP019725292, ISSN: 1437-7799, DOI: 10.1007/s10157-009-0162-x
- YOSHITAKA INOSAKA: "Recent advance in nephrotic syndrome", JOURNAL OF CLINICAL AND EXPERIMENTAL MEDICINE, vol. 252, no. 11 (3043), 14 March 2015 (2015-03-14), JP , pages 1166 - 1170, XP009538246, ISSN: 0039-2359
- KAWACHI H: "Therapeutic targets in podocyte", NEPHROLOGY, vol. 15, no. S3, 1 June 2010 (2010-06-01), pages 23 - 23, XP055950614, DOI: 10.1111/j.1440-1797.2010.01335.x
- YOSHIYASU FUKUSUMI, YING ZHANG, HIROSHI KAWACHI: "Vangl2 Is Associated with Ephrin-B1, a Novel Critical Component of the Slit Diaphragm, and Its Downregulation Is Involved in the Initiation Event of the Podocyte Injury", JOURNAL OF THE AMERICAN SOCIETY OF NEPHROLOGY, vol. 28, no. Suppl. 1, 1 January 2017 (2017-01-01) - 5 November 2017 (2017-11-05), pages 736, XP009538245
- SAYURI TAKAMURA, YOSHIYASU FUKUSUMI, YING ZHANG, ICHIEI NARITA, HIROSHI KAWACHI: "TH-PO069 Par6β Interaction with Ephrin-B1 at the Slit Diaphragm Could Be a Differential Diagnostic Marker of Nephrotic Syndrome: Interaction of the Par-Complex Molecules with Ephrin-B1/Nephrin in Podocyte", JOURNAL OF THE AMERICAN SOCIETY OF NEPHROLOGY, vol. 28, no. Suppl. 1, 1 January 2017 (2017-01-01) - 5 November 2017 (2017-11-05), US , pages 121, XP009538244, ISSN: 1046-6673

## Description

### [Technical Field]

The present invention relates to a marker for assisting in the diagnosis of nephrotic syndrome and use thereof. Specifically, the present invention relates to the use of Ephrin-B 1 as a marker in the diagnosis of nephrotic syndrome in a urine sample, and in a method for diagnosing nephrotic syndrome in a urine sample.

Priority is claimed on Japanese Patent Application No. 2021-004184, filed in Japan on January 14, 2021.

### [Background Art]

Currently, the number of potential patients with chronic kidney disease is estimated to be about 13 million, half of whom are believed to require treatment. As the kidney disease progresses, kidney failure occurs, in which kidney function is almost lost. Dialysis therapy such as hemodialysis (artificial dialysis) or kidney transplantation is the only treatment option for patients with kidney failure, and kidney transplantation is performed only in a limited number of cases, and most patients with kidney failure receive hemodialysis therapy. The number of patients receiving hemodialysis therapy exceeds 330,000, and the total medical expenses have reached approximately 2 trillion yen annually. Proteinuria is the most important aggravating factor for kidney disease progression. The development of a new drug that suppresses proteinuria is required because it has the effect of preventing or delaying the progression to kidney failure, and furthermore, leads to a reduction in the huge medical costs associated with dialysis therapy.

Regarding proteinuria, if the urinary protein is 3.5 g or more per day and the blood albumin concentration is 3.0 g/dL or less, it is diagnosed as nephrotic syndrome. Nephrotic syndrome is a disease in which a large amount of protein is excreted from the urine, resulting in a decrease in protein concentration in the blood (hypoproteinemia), and resulting in swelling (edema). In advanced nephrotic syndrome, fluid accumulates in the lungs, stomach, heart, and scrotum. In addition, hypoproteinemia increases cholesterol in the blood, and there is a risk of complications such as kidney failure, thrombosis (e.g., pulmonary infarction, myocardial infarction, cerebral infarction, etc.), infectious diseases, or the like.

The inventors clarified in their previous basic research that the slit membrane, which is the intercellular adhesion apparatus of the kidney glomerular epithelial cells (podocytes), functions as the final barrier to prevent proteinuria (see, for example, Non-Patent Document 1). In addition, the inventors identified molecules whose gene expression is changed during the pathogenesis period immediately before the onset of proteinuria by an analysis using a next-generation sequencer, examination using the Subtraction/Differential Display method, etc., and analyzed their expression patterns, localization and molecular functions, and searched and identified the molecular groups involved in the onset of proteinuria (see, for example, Non-Patent Document 1). A series of studies have revealed that Ephrin-B 1 is a membrane protein that constitutes the slit membrane and plays an important role in maintaining the barrier function of the slit membrane (see, for example, Non-Patent Document 2).

Non-Patent Document 2 shows that the expression of Ephrin-B1 is decreased prior to other functional molecules in filamentous epithelial cells in various disease induction animal models, and that the decrease in the expression (decreased function) of Ephrin-B1 is extremely important as an early change in the onset of pathology. However, the leakage of Ephrin-B 1 into urine and the role of Ephrin-B1 as a diagnostic marker have not yet been investigated. Non-patent Document D3 discloses Par6β interaction Ephrin-B1 at the slit diaphragm could be a differential diagnostic marker of nephrotic syndrome (PG121).
Non-patent Document D4 discloses proteinuria in several glomerular diseases resulting from the dysfunction of slit diaphragm such as minimal change nephrotic syndrome.

In addition, there is a demand for markers useful for pathological discrimination and prognostic diagnosis of nephrotic syndrome presenting with severe proteinuria.

### [Citation List]

### [Non-Patent Documents]

[Non-Patent Document 1] Miyauchi N et al., "Synaptic vesicle protein 2B is expressed in podocyte, and its expression is altered in proteinuric glomeruli", J Am Soc Nephrol., vol.17, p2748-2759, 2006.
[Non-Patent Document 2] Fukusumi Y et al., "Nephrin -Binding Ephrin-B 1 at the Slit Diaphragm Controls Podocyte Function through the JNK Pathway", J Am Soc Nephrol., vol. 29, Issue 5, pp. 1462-1474, 2018.
[Non-Patent Document 3] Sayuri Takamura et al., "TH-PO069 Par6[beta] interaction with Ephrin-B1 at the slit diaphragm could be a differential diagnostic marker of nephrotic syndrome: interaction of the Par-Complex molecules with Ephrin-B1/Nephrin in podocytes", Journal of the American Society of Nephrology ; Kidney week 2017 abstracts; New Orleans, LA / Oct. 31- Nov. 5, 2017, Williams and Wilkins, Baltimore, MD, US.
[Non-Patent Document 4] Sayuri Takamura et al., "Partitioning-defective-6-Ephrin-B1 interaction is regulated by nephrin-mediated signal and is crucial in maintaining slit diaphragm of podocyte", The American Journal of Pathology, vol. 190, no. 2, 1 Feb. 2020, pages 333-346.

### [Summary of Invention]

### [Technical Problem]

The present invention has been made in view of the above circumstances, and provides a novel marker molecule, Ephrin-B 1, useful for assisting in the diagnosis of nephrotic syndrome. A method using the marker molecule is also herein described.

### [Solution to Problem]

That is, the present invention includes the following aspects.
(1) Use of Ephrin-B1 as a marker for assisting in the diagnosis of nephrotic syndrome in a urine sample.
(2) A method for diagnosing nephrotic syndrome, comprising measuring the expression level of Ephrin-B 1 in the urine of a subject.

### [Advantageous Effects of Invention]

According to the above aspects, it is possible to provide a novel marker molecule useful for assisting in the diagnosis of nephrotic syndrome. This marker molecule is Ephrin-B 1.

### [Brief Description of Drawings]

FIG. 1 shows fluorescence images obtained by detecting the expression of Ephrin-B 1 in a normal (negative for proteinuria) human kidney specimen and a nephrotic syndrome human kidney specimen in Example 1.
FIG. 2 is a diagram showing the detection results of Ephrin-B1 in the urine of rats with adriamycin (ADR) nephropathy, which is a rat model of focal glomerulosclerosis in Example 2.
FIG. 3 is a diagram showing the detection results of Ephrin-B1 in the urine of rats with adriamycin (ADR) nephropathy, which is a rat model of focal glomerulosclerosis in Example 2.
FIG. 4 is a diagram showing the detection results of Ephrin-B1 in the urine of a mesangial proliferative nephritis rat model in Example 2.
FIG. 5 is a diagram showing the detection results of Ephrin-B1 in the urine of anti-nephrin antibody (ANA)-induced nephropathy (ANA nephropathy) rats, which is a slit membrane-specific disorder rat model in Example 2.
FIG. 6 is a diagram showing the detection results of Ephrin-B1 in the urine specimens provided from human patients with mild proteinuria in Example 3.
FIG. 7 is a diagram showing the detection results of Ephrin-B1 in the urine specimens at the time of remission (when proteinuria becomes mild) and at the time of relapse provided from human patients diagnosed with nephrotic syndrome in Example 4.

### [Description of Aspects]

### <Marker for assisting in diagnosis of nephrotic syndrome>

In one embodiment, the present invention provides the use of an Ephrin-B1 as a marker for assisting in the diagnosis of nephrotic syndrome.

In the present embodiment, Ephrin-B1 is useful as a marker to assist in the diagnosis of nephrotic syndrome.

The urine of healthy subjects contains approximately 0.15 g of urinary protein per day, and in the urinary protein test, if the urinary protein concentration is less than 15 mg/dL, it is regarded as no particular problem, and diagnosed as negative (-). On the other hand, in the urinary protein test, a urinary protein of 15 mg/dL or more and less than 30 mg/dL is diagnosed as a false positive (±), a urinary protein of 30 mg/dL or more and less than 100 mg/dL is diagnosed as positive (1+), a urinary protein of 100 mg/dL or more and less than 300 mg/dL is diagnosed as positive (2+), a urinary protein of 300 mg/dL or more and less than 1,000 mg/dL is diagnosed as positive (3+), and a urinary protein of 1,000 mg/dL or more is diagnosed as positive (4+).

In addition, in general, "nephrotic syndrome" is a disease in which a large amount of protein is excreted from urine, resulting in a decrease in protein concentration in the blood (hypoproteinemia), and resulting in swelling (edema). In addition, if the urinary protein is 3.5 g or more per day and the blood albumin concentration is 3.0 g/dL or less, it is diagnosed as nephrotic syndrome. In advanced nephrotic syndrome, fluid accumulates in the lungs, stomach, heart, and scrotum. In addition, hypoproteinemia increases cholesterol in the blood, and there is a risk of complications such as kidney failure, thrombosis (e.g., pulmonary infarction, myocardial infarction, cerebral infarction, etc.), infectious diseases, or the like.

As shown in the Examples below, Ephrin-B1 is detected in the urine even at the stage of mild proteinuria in various disease induction models. Therefore, Ephrin-B1 is useful as a marker for early diagnosis of nephrotic syndrome before it becomes severe.

Ephrin-B1 is a membrane protein that constitutes the slit membrane and plays an important role in maintaining the barrier function of the slit membrane. Therefore, Ephrin-B1 is useful as a pathological discrimination marker to determine whether the proteinuria (nephrotic syndrome) is caused by damage to the slit membrane.

Furthermore, as shown in the Examples described later, in various disease induction models, the amount of Ephrin-B1 detected in the urine tends to increase with an increase in the amount of protein in the urine, i.e., with the progression of nephrotic syndrome. Therefore, Ephrin-B1 is useful as a marker for diagnosing the progression of nephrotic syndrome or as a marker for prognosis.

### [Ephrin-B]

Ephrin-B is a single-span membrane protein that has a molecular weight of about 30 k to 45 k and is composed of an extracellular region, a transmembrane region, and an intracellular region having a PDZ-binding sequence at the C-terminus. It is expressed in kidney filamentous epithelial cells and is a protein that forms the slit membrane together with nephrin.

Ephrin-B is classified into three subclasses: Ephrin-B1, Ephrin-B2 and Ephrin-B3. Among them, and according to the claimed invention, it is preferable to use Ephrin-B1 as a marker because its expression is remarkable in kidney filamentous epithelial cells.

In the present specification, the use of Ephrin-B1 as a marker for assisting in the diagnosis of nephrotic syndrome includes the following aspects:
(1) Detecting or measuring the expression level of Ephrin-B1 in the urine.
(2) Detecting or measuring the presence of mRNA encoding Ephrin-B 1 or a nucleotide fragment having a partial sequence thereof in the urine.

To diagnose nephrotic syndrome by detecting the expression of Ephrin-B 1 in the urine or measuring the expression level, for example, a Western blotting method using a substance that specifically binds to the Ephrin-B1, an ELISA (Enzyme-Linked ImmunoSorbent Assay) method, a CLEIA (Chemiluminescent Enzyme Immuno Assay) method, immunological methods such as an immunoprecipitation method, an immunohistological staining method or the like, can be used.

The Western blotting method, for example, is a method in which a urine sample containing Ephrin-B is subjected to acrylamide ge1 electrophoresis to separate Ephrin-B, the separated Ephrin-B is transferred to a membrane and reacted with an antibody (primary antibody) capable of recognizing Ephrin-B, and a primary antibody that does not bind to Ephrin-B is removed by washing, etc., and then, the generated immune complex of Ephrin-B and the primary antibody is detected using a labeled secondary antibody. The amount of Ephrin-B present can be determined by measuring the amount of labeling of the labeled secondary antibody bound to the generated immune complexes.

In the immunohistological staining method, tissue sections, cells, etc. fixed on a slide glass are reacted with antibodies to generate immune complexes, and primary antibodies that are not bound to Ephrin-B are removed by washing, etc., and then detected by a labeled secondary antibody bound to the immune complex. The immunohistological staining method is effective for analyzing the expression site of Ephrin-B in tissues and cells.

Examples of the substance that specifically binds to the Ephrin-B, such as specific antibodies to the Ephrin-B (hereinafter sometimes referred to as "Ephrin-B-specific antibody"), include those exemplified in <Diagnostic reagent for nephrotic syndrome> described below.

In addition, the use of Ephrin-B1 as a marker for assisting in the diagnosis of nephrotic syndrome also includes methods of detecting a nucleotide fragment having an mRNA encoding Ephrin-B1, or a partial sequence thereof. More specific examples include, but are not limited to, quantitative RT-PCR, Northern blotting, in situ hybridization, and the like.

In such a detection method, probes and primers prepared based on known nucleotide sequence information of Ephrin-B1, or a combination thereof can be used.

In addition, for the mRNA encoding Ephrin-B 1 contained in the urine, or a nucleotide fragment having a partial sequence thereof, the presence of a polynucleotide encoding Ephrin-B1 can be detected by checking for amplification in a polymerase chain reaction (PCR) using specific primers.

In the present specification, the diagnostic target using the marker for assisting in the diagnosis of nephrotic syndrome of Ephrin-B1 is not particularly limited as long as it is an animal that develops nephrotic syndrome, but mammals are preferred. Examples of the mammals include, but are not limited to, humans, mice, rats, hamsters, guinea pigs, rabbits, dogs, cats, horses, cows, sheep, pigs, goats, marmosets, and monkeys. Among them, humans are preferable.

### <Method for examining nephrotic syndrome>

In one embodiment, the present invention provides a method for diagnosing nephrotic syndrome, comprising the step of measuring the expression level of an Ephrin-B 1 in the urine of a subject.

According to the diagnosing method (method for obtaining examination data) of the present embodiment, it is possible to diagnose whether or not the subject has a disease condition leading to nephrotic syndrome.

### [Measurement process]

Examples of the method for measuring the expression level of Ephrin-B1 contained in the urine obtained from a subject include, for example, a Western blotting method using a substance that specifically binds to the Ephrin-B1, an ELISA (Enzyme-Linked ImmunoSorbent Assay) method, a CLEIA (Chemiluminescent Enzyme Immuno Assay) method, and immunological methods such as immunoprecipitation.

The details of the Western blotting method are as described in <Marker for assisting in diagnosis of nephrotic syndrome> above.

Examples of the substance that specifically binds to the Ephrin-B1, such as Ephrin-B1-specific antibodies, include those exemplified in <Diagnostic reagent for nephrotic syndrome> described below.

Examples of the substance that specifically binds to the Ephrin-B1 include a method in which a substance that specifically binds to the Ephrin-B 1 is immobilized on a support, and a urine sample collected from a subject is added thereto and incubated to bind an Ephrin-B1 with the substance that specifically binds to the Ephrin-B 1, followed by washing and detecting the Ephrin-B 1 bound to the support via the substance that specifically binds to the Ephrin-B1 to detect the Ephrin-B 1 in the urine, and the like.

In the method for measuring Ephrin-B1, a control sample may be placed in addition to the urine sample of a subject whose Ephrin-B 1 is to be detected. Examples of the control sample include a negative control sample that does not contain an Ephrin-B1, a positive control sample that contains an Ephrin-B1, and the like. In this case, it is possible to detect Ephrin-B1 in the urine sample of a subject by comparing the result obtained with a negative control sample without Ephrin-B1 to the result obtained with a positive control sample with an Ephrin-B1. In addition, it is also possible to quantitatively detect Ephrin-B1 in the subject's urine based on the numerical value of the subject's urine sample and a standard curve. The standard curve is prepared in such a manner that a series of control samples with stepwise changes in concentration are prepared, and the detection result for each control sample is obtained as a numerical value to create a standard curve.

In addition, examples of the method for measuring the expression level of Ephrin-B1 contained in the urine obtained from a subject include, a method for detecting mRNA encoding Ephrin-B1, or a nucleotide fragment having a partial sequence thereof, and the like. More specific examples include, but are not limited to, quantitative RT-PCR, Northern blotting, in situ hybridization, and the like.

In such a detection method, probes and primers prepared based on known nucleotide sequence information of Ephrin-B1, or a combination thereof can be used.

In the examination method of the present embodiment, it is determined that the subject has nephrotic syndrome or is likely to develop nephrotic syndrome based on the measured expression level of Ephrin-B 1.

Specifically, when the expression level of Ephrin-B1 is greater than a preset reference value, it can be evaluated or predicted that the subject has nephrotic syndrome or is likely to develop nephrotic syndrome. On the other hand, when the protein expression level is equal to or less than a preset reference value, it can be evaluated or predicted that the subject does not have nephrotic syndrome or has not developed nephrotic syndrome. Therefore, the examination method of the present embodiment can also be said to be a method for diagnosing nephrotic syndrome. Alternatively, in the case where the subject has urinary protein but is less than 3.5 g per day and has not been diagnosed with nephrotic syndrome, the examination method of the present embodiment can also be said to be an early diagnosis method for nephrotic syndrome.

The reference value here is a reference value for distinguishing between a nephrotic syndrome patient group and a non-nephrotic syndrome patient group.

Alternatively, the reference value is a reference value for distinguishing between a nephrotic syndrome patient group caused by slit membrane disorder and a nephrotic syndrome patient group caused by something other than slit membrane disorder. When this reference value is used, the examination method of the present embodiment can also serve as a pathological discrimination method of nephrotic syndrome to discriminate whether or not the nephrotic syndrome is caused by slit membrane disorder.

The reference value can be determined experimentally as a threshold that can distinguish between the nephrotic syndrome patient group and the non-nephrotic syndrome patient group, for example, by measuring the Ephrin-B 1 concentrations in the urine of the two groups. Alternatively, the reference value can be determined experimentally as a threshold that can distinguish between the nephrotic syndrome patient group caused by slit membrane disorder and the nephrotic syndrome patient group caused by something other than slit membrane disorder, for example, by measuring the Ephrin-B 1 concentrations in the urine of the two groups.

The method for determining the reference value for the Ephrin-B 1 concentration in the urine in the present embodiment is not particularly limited, and can be determined, for example, using a general statistical method.

Specifically, in the method for determining the reference value, for example, the Ephrin-B 1 concentration in the urine collected in advance before the diagnosis based on the urinary protein concentration (at the time of hospitalization, etc.) is measured for patients who have been diagnosed as having nephrotic syndrome by other methods such as a commonly performed diagnosis by testing the amount of protein in the urine. After measuring a plurality of patients, the mean value or the median value of the Ephrin-B 1 concentrations in the urine is calculated, and a numerical value including the value can be used as the reference value.

Alternatively, for example, the Ephrin-B 1 concentration in the urine collected in advance before the diagnosis using a kidney specimen (at the time of hospitalization or the like) is measured for patients who have been diagnosed with nephrotic syndrome caused by slit membrane disorder by other methods such as a commonly performed diagnosis using kidney specimens. After measuring a plurality of patients, the mean value or the median value of the Ephrin-B1 concentration in the urine is calculated, and a numerical value including the value can be used as the reference value.

In addition, the reference value can be determined in such a manner that the Ephrin-B1 concentration in the urine collected in advance before the diagnosis based on the urinary protein concentration is measured (at the time of hospitalization, etc.) for a plurality of nephrotic syndrome patients and a plurality of non-nephrotic syndrome patients, and the Ephrin-B 1 concentrations in the urine of the nephrotic syndrome patient group and the non-nephrotic syndrome patient group and their variation are calculated from the mean value, the median value, or the like, followed by determining a threshold value that allows both values to be distinguished in consideration of the variations, as a reference value.

Alternatively, the reference value can be determined in such a manner that the Ephrin-B1 concentrations in the urine collected in advance before the diagnosis using a kidney sample are measured (at the time of hospitalization, etc.) for a plurality of patients with nephrotic syndrome caused by slit membrane disorder and a plurality of patients with nephrotic syndrome caused by something other than slit membrane disorder, and the mean value, the median value or the like is used to calculate the Ephrin-B1 concentrations in the urine of the nephrotic syndrome patient group caused by slit membrane disorder and the nephrotic syndrome patient group caused by something other than slit membrane disorder and their variation, followed by determining a threshold value that distinguishes the two numerical values is obtained in consideration of the variation. This threshold value is used as the reference value.

Further, for example, when the expression level of Ephrin-B 1 in the urine of a subject who is a nephrotic syndrome patient is higher than the expression level of Ephrin-B 1 in the urine at the time of the previous measurement of the subject, it can be evaluated or predicted that nephrotic syndrome is progressing or aggravated. Alternatively, when the expression level of Ephrin-B1 in the urine of a subject who is a nephrotic syndrome patient is lower than the expression level of Ephrin-B 1 in the urine at the time of the previous measurement of the subject, it can be evaluated or predicted that the symptoms of nephrotic syndrome tend to improve or that the nephrotic syndrome tends to recover. Therefore, the diagnosing method of the present embodiment can also be said to be a method of predicting the progression of nephrotic syndrome. Alternatively, the diagnosing method of the present embodiment can also be said to be a prognostic method for patients with nephrotic syndrome.

Moreover, the necessity of treatment can be determined according to the results of the diagnosing method of the present embodiment. That is, when the expression level of Ephrin-B 1 exceeds a preset reference value, treatment such as administration of medication to subjects is performed. In addition, the options of treatment for nephrotic syndrome can be selected.

As the treatment method for nephrotic syndrome, drug therapy using adrenal corticosteroids, immunosuppressants, renin-angiotensin-aldosterone system inhibitors such as angiotensin II receptor blockers and angiotensin-converting enzyme inhibitors, etc., alone or in combination of two or more, is mainly used. The dosage and period of use are appropriately set according to the symptoms and disease state of the subject. In addition, in treatment-resistant cases, biological agents (rituximab, etc.) and LDL apheresis therapy are used.

### <Diagnostic reagent for nephrotic syndrome>

### [First aspect]

In one aspect, the present description provides a diagnostic reagent (disclosed but not claimed) for nephrotic syndrome including a substance that specifically binds to the Ephrin-B.

According to the diagnostic reagent of the present aspect, it is possible to diagnose whether or not a subject has nephrotic syndrome. In addition, the diagnostic reagent of the present aspect is useful for early diagnosis of nephrotic syndrome before it becomes severe, diagnosis of progression of pathology, pathological discrimination, prognosis, and the like.

The substance that specifically binds to the Ephrin-B 1 herein described may be one type or two or more types, as long as the expression level of Ephrin-B1 can be measured.

Examples of the substance that specifically binds to the Ephrin-B1 include, Ephrin-B1-specific antibodies, aptamers, Ephs that are ligands for Ephrin-B1, and the like. Among them, Ephrin-B1-specific antibodies or Ephs are preferable.

The Ephrin-B1-specific antibodies may be polyclonal antibodies, monoclonal antibodies, and functional fragments of antibodies. Among them, the Ephrin-B1-specific antibody is preferably a monoclonal antibody because of its high specificity and excellent quantification.

The Ephrin-B1-specific antibodies may be commercially available products, or may be produced using the method described below. Examples of the commercially available Ephrin-B1-specific antibody include rabbit polyclonal anti-human Ephrin-B1 antibody (isotype: IgG) manufactured by Santa Cruz Biotechnology, Abcam's rabbit polyclonal anti-human Ephrin-B1 antibody (isotype: IgG), goat polyclonal anti-mouse Ephrin-B 1 antibody (isotype: IgG) manufactured by R&D Systems, mouse monoclonal anti-human Ephrin-B1 antibody (isotype: IgG) manufactured by Santa Cruz Biotechnology, mouse monoclonal anti-human Ephrin-B1 antibody (isotype: IgG) manufactured by Thermo Fisher Scientific, rabbit polyclonal anti-Ephrin-B antibody (isotype: IgG) manufactured by GeneTex, rabbit polyclonal anti-Ephrin-B antibody (isotype: IgG) manufactured by Proteintec, rabbit monoclonal anti-Ephrin-B antibody (isotype: IgG) manufactured by Cell Signaling Technology, and the like.

As used herein, the term "specifically bind" means that the antibody binds only to the target protein (antigen), and for example, it can be quantified by binding of an antigen in an in vitro assay, preferably a plasmon resonance assay using purified wild-type antigens (eg, BIAcore, GE-Healthcare Uppsala, Sweden, etc.). Binding affinity can be defined by ka (rate constant for antibody binding from an antibody-antigen complex), kD (dissociation constant), and KD (kD/ka). The binding affinity (KD) when the antibody specifically binds to the antigen is preferably 10⁻⁸ mol/L or less, and more preferably 10⁻¹³ mol/L or more and 10⁻⁹ mol/L or less.

In the present embodiment, the term "polyclonal antibody" refers to an antibody preparation including different antibodies directed against different epitopes. That is, when the antibody of the present embodiment is a polyclonal antibody, it may contain different antibodies that specifically bind to Ephrin-B 1.

In addition, the term "monoclonal antibody" refers to an antibody (including antibody fragments) obtained from a substantially homogeneous population of antibodies.

Furthermore, monoclonal antibodies, in contrast to polyclonal antibodies, refer to those that recognize a single determinant on an antigen.

In the present embodiment, the term "functional fragment" of an antibody means a portion (partial fragment) of an antibody that specifically recognizes a target protein. Specifically, Fab, Fab', F(ab')2, variable region fragment (Fv), disulfide bond Fv, single chain Fv (scFv), sc(Fv)2, diabodies, multispecific antibodies, polymers thereof, and the like can be mentioned.

Ephs are known as ligands for Ephrins. Although Eph is not particularly limited as long as it binds to the Ephrin-B1, EphB is preferable. Six subclasses of EphB have been identified: EphB1, EphB2, EphB3, EphB4, EphB5, and EphB6. The above EphB subclasses may be used alone, or in combination of two or more. Among the above EphB subclasses, EphB2 is preferable because it has the strongest binding to Ephrin-B 1.

Ephs may be commercially available products, chemically synthesized products, or those produced by known genetic engineering means based on the known base sequence information of Ephrin-B1. Examples of the commercially available Ephs include mouse EphB2-Fc chimeric recombinant protein and mouse EphB4-Fc chimeric recombinant protein manufactured by R&D Systems; human Fc tag-bound human EphB4 protein manufactured by Sino Biological; mouse EphB2-Fc chimeric recombinant protein, rat EphB1-Fc chimeric recombinant protein manufactured by Sigma; mouse EphB2-Fc chimeric recombinant protein, human EphB2-Fc chimeric recombinant protein, rat EphB1-Fc chimeric recombinant protein manufactured by Novus; and the like.

An aptamer is a substance that has specific binding ability to a target substance. Examples of the aptamer include nucleic acid aptamers, peptide aptamers and the like. Nucleic acid aptamers having specific binding ability to an antigen can be selected by, for example, the systematic evolution of ligand by an exponential enrichment (SELEX) method. In addition, peptide aptamers having specific binding ability to an antigen can also be selected by, for example, the two-hybrid method using yeast.

In addition, the substance that specifically binds to the Ephrin-B1 may be bound to a labeling substance or a modifying substance.

Examples of the labeling substance for the antibody include stable isotopes, radioactive isotopes, fluorescent substances, enzymes, magnetic substances and the like. Among them, the labeling substance for the antibody is preferably a fluorescent substance or an enzyme because of its easy detection and high sensitivity. Ephrin-B1 can be detected and quantified simply and with high sensitivity by including a labeling substance in the substance that specifically binds to the Ephrin-B1.

Examples of the stable isotope include, but are not limited to, ¹³C, ¹⁵N, ²H, ¹⁷O, ¹⁸O^{.}

Examples of the radioisotope include, but are not limited to, ³H, ¹⁴C, ¹³N, ³²P, ³³P, ³⁵S.

Examples of the fluorescent substance include, but are not limited to, cyanine dyes (eg, Cy3, Cy5, etc.), rhodamine 6G reagents, and other known fluorescent dyes (eg, GFP, FITC (Fluorescein), TAMRA, etc.).

Examples of the enzyme include an alkaline phosphatase and a peroxidase (HRP). When the labeling substance is an enzyme, it is preferable to use an enzyme substrate. As the enzyme substrate, in the case of alkaline phosphatase, p-nitrophenyl phosphate (pNPP), 4-methylumbelliferyl phosphate (4-MUP), etc. can be used, and if the enzyme is peroxidase, 3,3'-diaminobenzidine (DAB), 3,3',5,5'-tetramethylbenzidine (TMB), o-phenylenediamine (OPD), 2,2-azino-di-(3-ethylbenzothiazoline-6-sulfonic acid) (ABTS), 10-acetyl-3,7-dihydroxyphenoxazine (ADHP) and the like can be used.

Examples of the magnetic substance include a gadolinium, Gd-DTPA, Gd-DTPA-BMA, Gd-HP-DO3A, iodine, iron, iron oxide, chromium, manganese, or complexes or chelate complexes thereof, and the like, but are not limited to these.

Examples of the modifying substance include a biotin, Fc fragments and the like. By including the modifying substance in the substance that specifically binds to the Ephrin-B1, it is possible to detect and quantify Ephrin-B1 simply and highly sensitively using a specific binding substance for the modifying substance to which the labeling substance is bound (for example, a specific antibody, or avidin or streptavidin if it is a specific binding substance for biotin).

### (Method for producing antibody) (disclosed but not claimed)

When the Ephrin-B1-specific antibody is a polyclonal antibody, the antibody can be obtained by immunizing an immunized animal with an antigen (e.g., Ephrin-B1polyclonal antibody, or fragments thereof, or cells expressing these, etc.) and purifying the antisera by conventional means (e.g., salting out, centrifugation, dialysis, column chromatography, etc.).

Ephrin-B1 used as an antigen, or a peptide fragment having a partial amino acid sequence thereof, may be chemically synthesized, or may also be produced by known genetic engineering means based on the known base sequence information of Ephrin-B 1.

Moreover, when the Ephrin-B1-specific antibody is a monoclonal antibody, it can be produced by the hybridoma method or the recombinant DNA method.

Examples of the hybridoma method include Kohler and Milstein's method (see, for example, Kohler & Milstein, Nature, 256:495 (1975)) and the like. Examples of the antibody-producing cells used in the cell fusion step in this method include spleen cells, lymph node cells, peripheral blood leukocytes, etc. of animals (e.g. mice, rats, hamsters, rabbits, monkeys, goats, etc.) immunized with the antigen (Ephrin-B1 or fragments thereof, or cells expressing these, etc.). In addition, antibody-producing cells obtained by allowing an antigen to act in a culture medium on the above-mentioned cells or lymphocytes previously isolated from non-immunized animals can also be used. Various known cell lines can be used as myeloma cells. The antibody-producing cells and the myeloma cells may originate from different animal species as long as they can be fused, but preferably originate from the same animal species. Examples of the method for obtaining the hybridoma include a method in which cell fusion between spleen cells obtained from a mouse immunized with an antigen and mouse myeloma cells is performed, followed by screening to produce a monoclonal antibody specific to the target protein, or the like. Examples of the method for obtaining a monoclonal antibody produced by hybridomas include a method in which a monoclonal antibody against the target protein is obtained by culturing hybridomas or obtained from ascitic fluid of mammals to which hybridomas have been administered.

Examples of the recombinant DNA method include a method in which a DNA encoding the above antibody or a functional fragment of the antibody is cloned from a hybridoma, B cell, or the like, incorporated into an appropriate vector, and transferred to a host cell (e.g., mammalian cell lines, E. coli, yeast cells, insect cells, plant cells, etc.) to produce the antibody of the present aspect as a recombinant antibody (for example, PJ Delves, Antibody Production: Essential Techniques, 1997 WILEY, P.I. Shepherd and C. Dean Monoclonal Antibodies, 2000 OXFORD UNIVERSITY PRESS, Vandamme AM et al., Eur. J. Biochem. 192:767-775 (1990)).

In the expression of antibody-encoding DNA, host cells may be transformed by inserting DNAs encoding the heavy or light chains separately into expression vectors, and may also be transformed with the DNAs encoding the heavy and light chains incorporated into a single expression vector (e.g., International Patent Application No. 94/11523). The antibody of the present aspect can be obtained in a substantially pure and homogeneous form by culturing the host cells, and separating and purifying from the culture medium or inside the host cells. Antibody isolation and purification can be carried out using methods commonly used in the polypeptide purification. In the method using transgenic animal production technology, for example, a transgenic animal (e.g., cow, goats, sheep, pigs, etc.) into which an antibody gene is incorporated is produced, and a large amount of monoclonal antibody derived from the antibody gene is obtained from the milk of the transgenic animal.

The antibody may be an amino acid sequence variant as long as it can specifically bind to Ephrin-B1.

The amino acid sequence variant may be produced by mutagenesis into the DNA encoding the antibody chains, or by peptide synthesis. The site where the amino acid sequence of the antibody is modified may be the constant region of the heavy or light chain of the antibody, or may be the variable region (framework region and CDRs) as long as it has the same activity as the antibody before modification. Alternatively, a method of screening for an antibody with increased affinity for an antigen by modifying the CDR amino acids may also be used (e.g., PNAS, 102: 8466-8471 (2005), Protein Engineering, Design & Selection, 21: 485-493 (2008), International Publication No. 2002/051870, J. Biol. Chem., 280: 24880-24887 (2005 ), Protein Engineering, Design & Selection, 21: 345-351 (2008)).

The number of amino acids to be modified is preferably 10 amino acids or less, more preferably 5 amino acids or less, and most preferably 3 amino acids or less (e.g., 2 amino acids or less, 1 amino acid). The amino acid modification is preferably a conservative substitution.

In the present specification, the term "conservative substitution" means substitution with another amino acid residue having a chemically similar side chain. The groups of amino acid residues having a chemically similar amino acid side chain are well known in the art to which this invention pertains. For example, they can be classified into acidic amino acids (aspartic acid and glutamic acid), basic amino acids (lysine, arginine, histidine), and neutral amino acids, and the neutral amino acids can be classified into amino acids with hydrocarbon chains (glycine, alanine, valine, leucine, isoleucine, proline), amino acids with hydroxy groups (serine, threonine), sulfur-containing amino acids (cysteine, methionine), amino acids with amide groups (asparagine, glutamine), amino acids with imino group (proline), amino acids with aromatic groups (phenylalanine, tyrosine, tryptophan), or the like. The amino acid sequence variant preferably has a higher antigen-binding activity than the control antibody.

In the present aspect, the antigen-binding activity of the antibody (including the functional fragments of the above-described antibodies, amino acid sequence variants, etc.) can be evaluated by, for example, ELISA, Western blotting, immunoprecipitation, immunostaining, and the like.

### (Labeling reagent) (disclosed but not claimed)

The diagnostic reagent for nephrotic syndrome of the present aspect may contain, in addition to the substance that specifically binds to the Ephrin-B1, a substance in which a labeled substance is bound to the substance that specifically binds to the Ephrin-B1 (e.g., a substance in which a labeled substance is bound to the Ephrin-B1-specific antibody), or a substance in which a labeled substance is bound to an antibody against the substance that specifically binds to the Ephrin-B1 (e.g., a substance in which a labeled substance is bound to an antibody against the Ephrin-B1-specific antibody), as a labeled reagent.

For example, when an Ephrin-B1-specific antibody to which a labeling substance is bound is provided as a labeling reagent, the specific Ephrin-B 1 can be detected and quantified using a sandwich ELISA (Enzyme-Linked ImmunoSorbent Assay) method, a chemiluminescence enzyme immunoassay method (CLEIA method) using an antigen measurement system, or the like.

Further, for example, when an antibody to the Ephrin-B1-specific antibody, to which a labeling substance is bound, is provided as a labeling reagent, the specific Ephrin-B1 can be detected and quantified using an ELISA method using an antibody measurement system, an indirect fluorescent antibody method, a CLEIA method using an antibody measurement system, or the like.

The antibody against the Ephrin-B1-specific antibody is preferably an antibody against the animal from which the Ephrin-B1-specific antibody is derived. For example, when the Ephrin-B1-specific antibody is derived from a mouse, it is preferably an anti-mouse antibody. In addition, the substance obtained by binding the labeled substance to the antibody against the Ephrin-B1-specific antibody includes all immunoglobulin classes and subclasses.

The diagnostic reagent for nephrotic syndrome of the present aspect may further include a reaction-stopping solution, if necessary. Examples of the reaction-stopping solution include sulfuric acid, sodium hydroxide and the like.

The diagnostic reagent for nephrotic syndrome of the present aspect may further include a buffer. The buffer can be appropriately selected from those known in the art, and examples thereof include a phosphate buffer, Tris-HCl buffer, citrate buffer, carbonate buffer, borate buffer, succinate buffer, acetate buffer and the like. The buffer may contain at least one selected from the group consisting of NaCl, a surfactant (e.g., Tween 20, Triton X-100, etc.) and a preservative (e.g., sodium azide, etc.), if necessary. Specific examples of the buffer include a phosphate-buffered saline (PBS), PBS-T (PBS-Tween 20), Tris-buffered saline (TBS), TBS-T (TBS-Tween 20) and the like.

In the diagnostic reagent for nephrotic syndrome of the present aspect, the substance that specifically binds to the Ephrin-B1 (specific antibody, Eph, etc.) and the labeling reagent may be in a dry state or dissolved in the above-described buffer. Among them, these antibodies are preferably in a dry state from the viewpoint of storage stability.

### [Second aspect]

In one aspect, the present description provides a diagnostic reagent (disclosed but not claimed) for nephrotic syndrome including a random primer for a reverse transcription reaction and a forward and reverse primer for amplifying the cDNA encoding Ephrin-B 1 among the reverse transcription products obtained by the reverse transcription reaction using the random primer.

The diagnostic reagent for nephrotic syndrome of the present aspect can be used when measuring the expression level of the gene encoding Ephrin-B1 (i.e., the expression level of mRNA) by a quantitative RT-PCR method. According to the diagnostic reagent for nephrotic syndrome of the present aspect, it is possible to easily and accurately diagnose whether or not a subject has nephrotic syndrome.

Examples of the random primer for the reverse transcription reaction in the present aspect include those composed of a mixture of 6-mer or 9-mer deoxyribonucleotides having random sequences, or the like, and the 5' end of the deoxyribonucleotides is preferably phosphorylated.

The forward primer in the present aspect can be a sequence specific to the nucleotide sequence of the 3' end region of the cDNA derived from the mRNA encloding Ephrin-B 1 among the reverse transcription products obtained by the random primers for the reverse transcription reaction. Further, the reverse primer in the present aspect can be a sequence specific to the nucleotide sequence of the 5' end region of the cDNA derived from the mRNA encoding Ephrin-B1 among the reverse transcription products obtained by the random primers for the reverse transcription reaction. Since the forward primer and the reverse primer have the above sequences, the cDNA encoding Ephrin-B 1 can be more specifically amplified in the real-time PCR reaction among the reverse transcription products obtained by the reverse transcription reaction using the random primers.

The nucleotide sequences of forward and reverse primers for amplifying cDNA derived from mRNA encoding human Ephrin-B 1 (claimed) or B2 (disclosed but not claimed) are shown in Table 1 below. The nucleotide sequence of mRNA encoding the human Ephrin-B 1 is disclosed in Genbank Accession No. NM_004429.5, and the nucleotide sequence of mRNA encoding the human Ephrin-B2 is disclosed in Genbank Accession No. NM_004093.4. The forward primer and the reverse primer for amplifying cDNA derived from the mRNA encoding the human Ephrin-B1 or Ephrin-B2 are not limited to those listed in Table 1 below, and can be appropriately designed by those skilled in the art based on the nucleotide sequence of mRNA of the above Genbank Accession numbers.

**[Table 1]**

| | Primer | Nucleotide sequence (5'→3') | Sequence number |
|---|---|---|---|
| Ephrin-B 1 | Forward | 5'-GGCAAGCATGAGACTGTGAA-3' | 1 |
| | Reverse | 5'-CCGTAAGGGAATGATGATGT-3' | 2 |
| Ephrin-B2 | Forward | 5'-TCCAACAAGACGTCCAGAGC-3' | 3 |
| | Reverse | 5'-AGAACAAGGTGCGAGTTCCC-3' | 4 |

The diagnostic reagent for nephrotic syndrome of the present aspect may further include a labeled oligonucleotide probe. The labeled oligonucleotide probe contains a nucleotide sequence complementary to at least three bases of the nucleotide sequence of the cDNA derived from the mRNA encoding Ephrin-B1. By including the nucleotide sequence, the probe can specifically hybridize to the amplification product of the forward primer and the reverse primer. As the labeled probe in the present aspect, for example, those having a quencher bound to the 5' end and a labeling substance bound to the 3' end can be used. By providing a quencher at the 5' end and a labeling substance at the 3' end, due to the 5'-3' exonuclease activity of the polymerase during the elongation reaction by the DNA polymerase in the real-time PCR reaction, among the reverse transcription products, the probe hybridized with the cDNA derived from the mRNA encoding Ephrin-B is degraded, and suppression by the quencher is released, so that the labeling substance can be detected.

Examples of the labeling substance bound to the 3' end of the probe include fluorescent dyes, fluorescent beads, quantum dots, biotin, antibodies, antigens, energy-absorbing substances, radioisotopes, chemiluminescers, enzymes and the like.

Examples of the fluorescent dye include FAM (carboxyfluorescein), JOE (6-carboxy-4',5'-dichloro 2',7'-dimethoxyfluorescein), FITC (fluorescein isothiocyanate), TET (tetrachlorofluorescein), HEX (5'-hexachloro-fluorescein-CE phosphoramidite), Cy3, Cy5, Alexa568, Alexa647 and the like.

When the labeling substance bound to the 3' end of the probe is a fluorescent dye, the combination of the labeling substance bound to the 3' end of the probe and the quencher bound to the 5' end of the probe is preferably a combination of labeling substances capable of causing FRET (fluorescence resonance energy transfer; Fluorescence (Foerster) Resonance Energy Transfer). Specific examples include a combination of a fluorescent dye having an excitation wavelength of around 490 nm (e.g., FITC, rhodamine green, Alexa (registered trademark) fluor 488, BODIPY FL, etc.) and a fluorescent dye having an excitation wavelength of around 540 nm (e.g., TAMRA, Tetra methylrhodamine, Cy3), or a combination of a fluorescent dye having an excitation wavelength of around 540 nm and a fluorescent dye having an excitation wavelength of around 630 nm (e.g., Cy5, etc.), and the like.

Although dependent on the nucleic acid amplification method, the diagnostic reagent for nephrotic syndrome of the present aspect may additionally contain one or more nucleotide triphosphates as substrates, nucleic acid synthase, and amplification reaction buffers. Nucleotide triphosphates are substrates (dNTPs, rNTPs, etc.) for nucleic acid synthetase. Nucleic acid synthetase is an enzyme according to the nucleic acid amplification method to be used, and examples thereof include a DNA polymerase, RNA polymerase, reverse transcriptase, and the like. Examples of the amplification reaction buffer include Tris buffer, phosphate buffer, Veronal buffer, borate buffer, Good's buffer and the like, and the pH is not particularly limited.

### [EXAMPLES]

The present invention will be described below with reference to Examples, but the present invention is not limited to the following Examples.

### [Example 1]

### (1) Sample preparation

With the permission of the donor, some of the samples obtained by kidney biopsy (examination method to pierce the kidney with a thin needle and take a tissue sample) in healthy subjects and patients with nephrotic syndrome were used as samples of glomerular epithelial cells. The obtained samples were immediately frozen at -70°C using n-hexane.

Next, a plurality of 3-µm-thick frozen sections were prepared from each of the healthy subject samples and the nephrotic syndrome patient-derived samples.

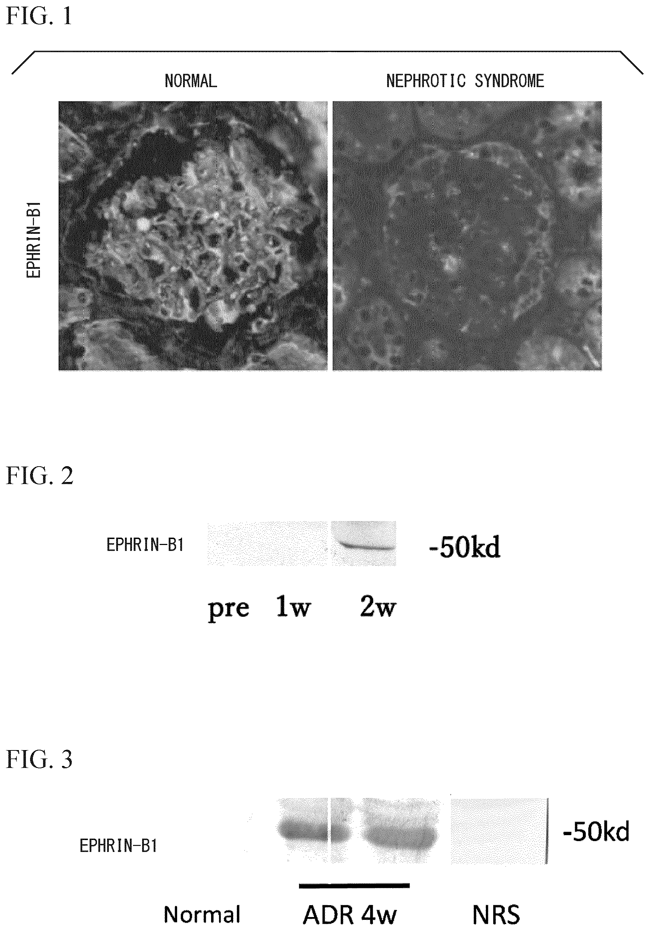

(2) Detection of Ephrin-B 1
The sections were then incubated at 4°C for 16 hours with an anti-Ephrin-B1 antibody (manufactured by Santa Cruz Biotechnology, rabbit anti-human Ephrin-B1 antibody). Then, after thorough washing, staining was performed using an FITC-conjugated anti-rabbit IgG antibody (manufactured by DAKO). Then, the sections were observed using an IF microscope (BX50, manufactured by Olympus) (magnification: 400 times). The results are shown in FIG.1. In FIG. 1, "Normal" indicates the sample of a healthy subject, and "Nephrotic syndrome" indicates the sample of a nephrotic syndrome patient.

As shown in FIG. 1, in the proteinuria-negative normal human kidney specimen, Ephrin-B 1 was observed in a linear pattern along the glomerular capillary wall. On the other hand, the expression of Ephrin-B1 was remarkably decreased in the human kidney specimens with nephrotic syndrome exhibiting severe proteinuria.

### [Example 2]

### (1) Preparation of various disease induction rat models of nephrotic syndrome

Various disease induction rat models of nephrotic syndrome were prepared using a known method.

As the various disease induction rat models of nephrotic syndrome, adriamycin (ADR) nephropathy rats, mesangial proliferative nephritis rats, and anti-nephrin antibody (ANA)-induced nephropathy (hereinafter, may be referred to as "ANA nephropathy") were used.

ADR nephropathy rats are a model that exhibits persistent and progressive proteinuria and eventually develops kidney failure. On the other hand, mesangial proliferative nephritis rats and ANA nephropathy show marked proteinuria (proteinuria equal to or greater than day 20 of ADR nephropathy) that peaks 10 days and 5 days after disease induction, respectively, but proteinuria normalizes 3 to 4 weeks after disease induction.

Specifically, rats with ADR nephropathy were intravenously injected with adriamycin (6.0 mg/1 kg body weight, dissolved in physiological saline) under anesthesia to induce ADR nephropathy. Urine samples from the rats before injection of adriamycin, and 1 week, 2 weeks and 4 weeks after injection of adriamycin were used. In addition, the test with urine samples before adriamycin injection and 1 week and 2 weeks after adriamycin injection and the test with urine samples 4 weeks after adriamycin injection were independent tests. The urine samples were collected in a metabolic gauge for 24 hours.

For the mesangial proliferative nephritis rats, the rats were given a single intravenous injection of monoclonal anti-Thy-1.1 antibody (mAb1-22-3, see Reference 1 (Ikezumi Y et al., "FK506 ameliorates proteinuria and glomerular lesions induced by anti-Thy 1.1 monoclonal antibody 1-22-3.", Kidney International, Vol. 61, pp. 1339 -1350, 2002.); 500 µg/rat; dissolved in physiological saline) under anesthesia to induce mesangial proliferative nephritis. Rat urine samples before injection of anti-Thy-1.1 antibody and 1, 3, 5, 7 and 10 days after injection of anti-Thy-1.1 antibody were used. The urine samples were collected in a metabolic gauge for 24 hours.

For the ANA nephropathy rats, the rats were given a single intravenous injection of monoclonal anti-nephrin antibody (mAb5-1-6, see Non-Patent Document 1 and Non-Patent Document 2; 10 mg/rat; dissolved in physiological saline) under anesthesia to induce ANA nephropathy. The rat urine samples 5 days after the injection of anti-nephrin antibody were used. The urine samples were collected in a metabolic gauge for 24 hours.

Then, using each urine sample obtained, Ephrin-B 1 was detected in the urine sample by Western blotting using an anti-Ephrin-B1 antibody (manufactured by Santa Cruz Biotechnology, rabbit anti-human Ephrin-B 1 antibody).

The results in ADR nephropathy rats are shown in FIG. 2 (after 2 weeks) and FIG. 3 (after 4 weeks). In FIG. 3, "Normal" is a normal rat urine sample without induced ADR nephropathy. "NRS" (Normal Rabbit Serum) is a negative control in which the urine samples from ADR nephropathy rats were reacted with the normal rabbit serum instead of antibodies.

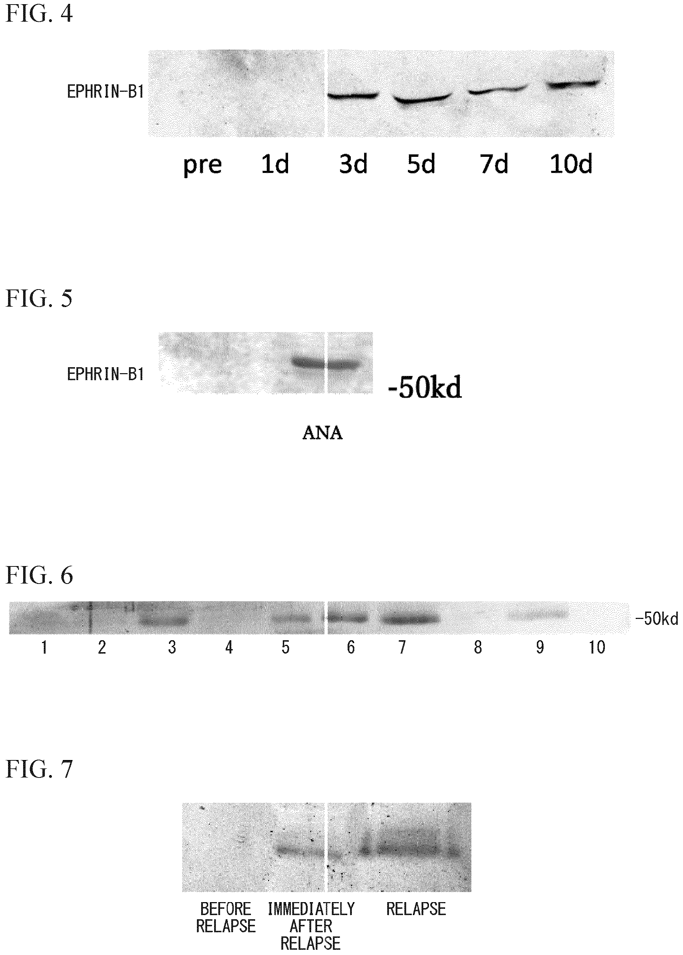

FIG. 4 shows the results in the mesangial proliferative nephritis rats.

FIG. 5 shows the results in the ANA nephropathy rats.

As shown in FIGS. 2 and 3, in the ADR nephropathy rats, Ephrin-B 1 was detected in the urine 2 weeks after the disease induction. In addition, 4 weeks after the disease induction, the expression of Ephrin-B1 in the urine was remarkable, and it was speculated that the detected amount of Ephrin-B 1 in the urine increased as the disease condition progressed. This suggests that Ephrin-B1 can be a useful marker for diagnosing the progression of nephrotic syndrome.

As shown in FIG. 4, in the mesangial proliferative nephritis rats, proteinuria peaked 10 days after the disease induction, whereas Ephrin-B 1 was already detected in the urine 3 days after the disease induction. This suggests that Ephrin-B1 can be a useful marker for early diagnosis of nephrotic syndrome before it becomes severe.

As shown in FIG. 5, it was also detected in the urine of ANA nephropathy rats 5 days after the disease induction.

### [Example 3]

With the permission of the donor, Ephrin-B1 was detected in the urine specimens (10 specimens) from human patients presenting with mild proteinuria on physical examination by Western blotting using an anti-Ephrin-B1 antibody (manufactured by R&D Systems, goat anti-Ephrin-B 1 antibody (isotype: IgG)). The results are shown in FIG. 6.

As shown in FIG. 6, Ephrin-B 1 was detected in some of the human urine specimens.

### [Example 4]

With the permission of the donor, Ephrin-B1 was detected in the urine specimens at the time of remission (when proteinuria becomes mild) and at the time of relapse provided from human patients diagnosed with nephrotic syndrome by Western blotting using the same antibody as in Example 3. The results are shown in FIG. 7.

As shown in FIG. 7, Ephrin-B 1 was detected in the human urine specimens immediately after recurrence of nephrotic syndrome and at the time of recurrence (when proteinuria became prominent).

### [Industrial applicability]

Inventors herein provide a novel marker molecule useful for assisting in the diagnosis of nephrotic syndrome. The examination method and diagnostic reagent for nephrotic syndrome herein described utilize the Ephrin-B 1 marker molecule, and are useful for diagnosing nephrotic syndrome in a urine sample, specifically, for example, early diagnosis, pathological discrimination and prognostic diagnosis.

### [Sequence listing]

PC33233_WA_sequence list.txt

## Claims

1. Use of Ephrin-B1 in a urine sample as a marker in the diagnosis of nephrotic syndrome.

2. A method for diagnosing nephrotic syndrome in a urine sample by using Ephrin-B 1 as a marker.

## Patentansprüche

1. Verwendung von Ephrin-B1 in einer Urinprobe als Marker in der Diagnose des nephrotischen Syndroms.

2. Ein Verfahren zur Diagnose des nephotischen Syndroms in einer Urinprobe unter Verwendung von Ephrin-B1 als Marker.

## Revendications

1. Utilisation dans un échantillon d'urine de l'éphrine B1 comme marqueur de diagnostic du syndrome néphrotique.

2. Méthode de diagnostic du syndrome néphrotique dans un échantillon d'urine utilisant l'éphrine B1 comme marqueur.
